# EUROPEAN PATENT APPLICATION

(11) **EP 3 816 299 A1**
(43) Date of publication of application: **05.05.2021**
(21) Application number: 19206425.1
(22) Date of filing: 31.10.2019
(51) Int. Cl.: C12Q 1/6806

(54) **A METHOD TO PREPARE PERSONALIZED TARGET-IRRELEVANT GUIDE RNA FOR CRISPR**

(71) Applicant: Siemens Healthcare GmbH, 91052 Erlangen (DE)
(72) Inventor: Huang, Yiwei, 91058 Erlangen (DE)
(74) Representative: Maier, Daniel Oliver

(57) **Abstract**

The present invention relates to a method of obtaining an enriched personalized population of a target polynucleotide using a synthetic single guide RNA (sgRNA) for an sgRNA-guided nucleic acid-binding protein, as well as to a method of obtaining a pool of personalized target-irrelevant synthetic single guide RNAs (sgRNAs) for a sgRNA-guided nucleic acid-binding protein. Also provided is a kit comprising a pool of sgRNAs obtainable by the methods of the invention, the use of a pool of sgRNAs obtainable by the methods of the invention and a method of monitoring a disease state.

## Description

### TECHNICAL FIELD

The present invention relates to a method of obtaining an enriched personalized population of a target polynucleotide using a synthetic single guide RNA (sgRNA) for an sgRNA-guided nucleic acid-binding protein, as well as to a method of obtaining a pool of personalized target-irrelevant synthetic single guide RNAs (sgRNAs) for a sgRNA-guided nucleic acid-binding protein. Also provided is a kit comprising a pool of sgRNAs obtainable by the methods of the invention, the use of a pool of sgRNAs obtainable by the methods of the invention and a method of monitoring a disease state.

### BACKGROUND

Next-generation sequencing (NGS) is a major driver in genetics and molecular research, including modern diagnostics inter alia in the field of cancer medicine. The technology provides a powerful way to study DNA or RNA samples. New and improved methods and protocols have been developed to support a diverse range of applications, including the analysis of genetic variations and sample specific differences. To improve this approach, methods have been developed that aim at a targeted enrichment of sequencing libraries by focusing on specific sequences, transcripts, genes or genome subregions, or by eliminating undesirable sequences.

Targeted enrichment can be useful in a number of situations where, for example, particular portions of a whole genome need to be analyzed. The efficient sequencing of a complete exome (all transcribed sequences) is a typical example for this approach. Further examples include the enrichment of specific transcripts, the enrichment of mutation hotspots or the exclusion of disturbing nucleic acid species. Such targeted enrichment strategies are, in particular, of importance in the context of personalized sequence determination or patient-based molecular monitoring, where (i) a library of a patient's transcriptome is to be analyzed and monitored and (ii) only a subset of the sequences in the library is of diagnostic relevance.

Current techniques for targeted enrichment include (i) Hybrid capture, wherein nucleic acid strands derived from the input sample are hybridized specifically to pre-prepared DNA fragments complementary to the targeted regions of interest, either in solution or on a solid support, so that one can physically capture and isolate the sequences of interest; (ii) Selective circularization or molecular inversion probes (MIPs), wherein single-stranded DNA circles that include target region sequences are formed by gap-filling and ligation chemistries in a highly specific manner, creating structures with common DNA elements that are then used for selective amplification of the targeted regions of interest; and (iii) Polymerase Chain Reaction (PCR) amplification, wherein PCR is directed toward the targeted regions of interest by conducting multiple long-range PCRs in parallel, a limited number of standard multiplex PCRs or highly multiplexed PCR methods that amplify very large numbers of short fragments (Mertes et al., 2011, Briefings in functional Genomics, 10, 6, 374-386).

More recently, the CRISPR/Cas-technology was used for targeted enrichment purposes, in particular in order to remove unwanted sequences from a sequencing library.

The CRISPR/Cas-technology is a new and very versatile genome- and epigenome-editing tool based on repurposing the CRISPR/Cas (clustered regularly interspersed short palindromic repeats / Cas) bacterial immune system (Cong et al, 2013, Science, 339, 819-824). The Cas nuclease, when complexed with a short RNA oligonucleotide known as a single guide RNA (sgRNA), can induce double-stranded breaks (DSBs) at specific sgRNA complementary locations.

The CRISPR/Cas system has further been repurposed as a programmable restriction enzyme to direct cleavage in a very precise and customized manner (Lee et al., 2015, Nucleic Acids Res., 43, 1-9).

Accordingly, methods using CRISPR/Cas have been developed which selectively deplete overabundant sequences in a process termed Depletion of Abundant Sequences by Hybridization (DASH). DASH was used to remove targets such as ribosomal RNA (rRNA) from mRNA-seq and wild-type KRAS background sequence from cancer samples by directing their targeted cleavage and preventing their further amplification and sequencing (Gu et al., 2016. Genome Biol., 17, 41). According to Gu et al., employing DASH after transposon-mediated fragmentation but prior to the following amplification step (which relies on the presence of adaptor sequences on both ends of the fragment) is capable of preventing amplification of the targeted sequences (mitochondrial rRNA), thereby ensuring they are not represented in the final sequencing library.

However, this enrichment approach is suitable to remove only a specific species from the sequencing library, while all other sequences remain in the library. There is hence a need for a versatile personalized transcriptome-based enrichment and analysis approach, which allows for an efficient reduction of the complexity of the sequencing library and thereby implicates a decreased sequencing depth and a manageable amount of data to be processed and thereby allows for repeated performance, e.g. in a monitoring approach.

### SUMMARY

The present invention addresses this need and provides a method of obtaining an enriched personalized population of a target polynucleotide comprising:(i) purifying a population of mRNA molecules from a sample obtained from a subject; (ii) preparing cDNA from the mRNA molecules of step (i); (iii) amplifying one or more target sequences from the cDNA obtained in step (ii) to obtain a pool of DNA molecules;(iv) fragmenting the amplified DNA molecules, preferably to a size of 20 to 30 bp; (v) connecting the fragments of step (iv) to a tag capable of binding to a cognate interactor to yield a pool of tagged catcher oligonucleotides; (vi) providing a pool of starting oligonucleotides for the preparation of a pool of synthetic single guide RNAs synthetic single guide RNA (sgRNA) for an sgRNA-guided nucleic acid-binding protein, wherein said starting oligonucleotide comprises a promoter segment, a random segment as potentially complementary sequence for the catcher oligonucleotide and a binding segment, which is complementary to at least a portion of a scaffold sequence for interaction with the sgRNA-guided nucleic acid-binding protein; (vii) hybridizing said pool of starting oligonucleotides and said tagged catcher oligonucleotide(s); (viii) removing complexes of starting oligonucleotides and tagged catcher oligonucleotides from said pool of starting oligonucleotides by binding said tag to a cognate interactor, preferably located on a bead or a suitable surface, thereby obtaining a reduced pool of starting oligonucleotides; (ix) preparing a pool of sgRNAs with said reduced pool of starting oligonucleotides obtained in step (viii); (x) cleaving a mixture of polynucleotides obtained from a test sample with an sgRNA-guided nucleic acid-binding protein using the pool of sgRNAs obtained in step (ix); and (xi) size selecting one or more uncut target polynucleotides from said mixture of polynucleotides obtained in step (x). The method advantageously allows to remove all sequences which are target-irrelevant with respect to the personalized target polynucleotide, i.e. gene or panel of genes of a patient's transcriptome, via the provision of an sgRNA pool capable of binding to said sequences. Thereby the complexity of the resulting personalized sequencing library is drastically reduced and performing sequencing operations, in particular next generation sequencing (NGS), on the library involves a much lower sequencing depth which significantly reduces the sequencing costs as well as subsequent data management and data processing costs.

In a further aspect the present invention relates to a method of obtaining a pool of personalized target-irrelevant synthetic single guide RNAs (sgRNAs) for a sgRNA-guided nucleic acid-binding protein comprising (i) purifying a population of mRNA molecules from a sample obtained from a subject; (ii) preparing cDNA from the mRNA molecules of step (i); (iii) amplifying one or more target sequences from the cDNA obtained in step (ii) to obtain a pool of DNA molecules; (iv) fragmenting the amplified DNA molecules, preferably to a size of 20 to 30 bp; (v) connecting the fragments of step (iv) to a tag capable of binding to a cognate interactor to yield a pool of tagged catcher oligonucleotides; (vi) providing a pool of starting oligonucleotides for the preparation of a pool of synthetic single guide RNAs synthetic single guide RNA (sgRNA) for an sgRNA-guided nucleic acid-binding protein, wherein said starting oligonucleotide comprises a promoter segment, a random segment as potentially complementary sequence for the catcher oligonucleotide and a binding segment, which is complementary to at least a portion of a scaffold sequence for interaction with the sgRNA-guided nucleic acid-binding protein; (vii) hybridizing said pool of starting oligonucleotides and said tagged catcher oligonucleotide(s); (viii) removing complexes of starting oligonucleotides and tagged catcher oligonucleotides from said pool of starting oligonucleotides by binding said tag to a cognate interactor, preferably located on a bead or a suitable surface, thereby obtaining a reduced pool of starting oligonucleotides; and (ix) preparing a pool of sgRNAs with said reduced pool of starting oligonucleotides obtained in step (viii).

In a preferred embodiment of the present invention, the amplification (iii) is performed as polymerase chain reaction (PCR) .

In a particularly preferred embodiment, the tag capable of binding to a cognate interactor is biotin and wherein said cognate interactor is streptavidin.

In a further embodiment the step of connecting the fragments to a biotin tag comprises an end-tailing with activated biotin, a ligation reaction with biotin or a linkage to biotin via click chemistry.

In yet another embodiment said the sgRNA-guided nucleic acid-binding protein is a DNA binding Cas protein, preferably a Cas9 protein or a derivative thereof.

In a further embodiment of the present invention, the random segment comprises between about 10 to 30 random nucleotides It is preferred that the random segment comprises about 20 random nucleotides.

In another embodiment of the present invention the steps (vii) to (viii) as mentioned above are repeated 1, 2, 3, 4, 5 or more times.

In another embodiment of the present invention said one or more target polynucleotides or target sequences represent a gene, one or more exons of a gene, and/or an open reading frame or a sub-portion thereof; or a panel of different genes, a panel of one or more exons of different genes, a and/or a panel of open reading frames or sub-portions thereof, or any combination of any of the before mentioned elements.

In yet another preferred embodiment of the present invention said method of obtaining an enriched personalized population of a target polynucleotide as described above additionally comprises as step (xii) a step of sequencing said size selected uncut target polynucleotide(s).

In another aspect the present invention relates to a kit comprising a pool of sgRNAs obtainable by the method of obtaining a pool of personalized target-irrelevant synthetic single guide RNAs (sgRNAs) for a sgRNA-guided nucleic acid-binding protein as described above and a sgRNA-guided nucleic acid-binding protein. It is preferred that the sgRNA-guided nucleic acid-binding protein is a Cas9 protein or derivative thereof.

In a further aspect the present invention relates to the use of a pool of sgRNAs obtainable by the method of obtaining a pool of personalized target-irrelevant synthetic single guide RNAs (sgRNAs) for a sgRNA-guided nucleic acid-binding protein as described above for the removal of target-irrelevant polynucleotides from a mixture of polynucleotides in a Cas9-based endonuclease assay.

In yet another aspect the present invention relates to a method of monitoring a disease state comprising the performance of the method of obtaining an enriched personalized population of a target polynucleotide as described above in a predefined interval of time. It is preferred that the method is performed according to the requirements of a treatment of said disease.

It is to be understood that the features mentioned above and those yet to be explained below may be used not only in the respective combinations indicated, but also in other combinations or in isolation without departing from the scope of the invention.

### BRIEF DESCRIPTION OF THE DRAWINGS

FIG. 1 shows a schematic illustration of the steps for obtaining a pool of personalized target-irrelevant synthetic single guide RNAs (sgRNAs) according to an embodiment of the present invention.
FIG. 2 depicts steps for the preparation of a target irrelevant sgRNA to be used according to an embodiment of the present invention.

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms " (i) ", "(ii)", " (iii) " or "(a)", "(b)", "(c)", "(d)", or "first", "second", "third" etc. and the like in the description or in the claims, are used for distinguishing between similar elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e. the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

As has been set out above, the present invention concerns in one aspect a method of obtaining an enriched personalized population of a target polynucleotide comprising: (i) purifying a population of mRNA molecules from a sample obtained from a subject; (ii)preparing cDNA from the mRNA molecules of step (i); (iii) amplifying one or more target sequences from the cDNA obtained in step (ii) to obtain a pool of DNA molecules; (iv) fragmenting the amplified DNA molecules, preferably to a size of 20 to 30 bp; (v) connecting the fragments of step (iv) to a tag capable of binding to a cognate interactor to yield a pool of tagged catcher oligonucleotides; (vi) providing a pool of starting oligonucleotides for the preparation of a pool of synthetic single guide RNAs synthetic single guide RNA (sgRNA) for an sgRNA-guided nucleic acid-binding protein, wherein said starting oligonucleotide comprises a promoter segment, a random segment as potentially complementary sequence for the catcher oligonucleotide and a binding segment, which is complementary to at least a portion of a scaffold sequence for interaction with the sgRNA-guided nucleic acid-binding protein; (vii) hybridizing said pool of starting oligonucleotides and said tagged catcher oligonucleotide(s); (viii) removing complexes of starting oligonucleotides and tagged catcher oligonucleotides from said pool of starting oligonucleotides by binding said tag to a cognate interactor, preferably located on a bead or a suitable surface, thereby obtaining a reduced pool of starting oligonucleotides; (ix) preparing a pool of sgRNAs with said reduced pool of starting oligonucleotides obtained in step (viii);(x) cleaving a mixture of polynucleotides obtained from a test sample with an sgRNA-guided nucleic acid-binding protein using the pool of sgRNAs obtained in step (ix); and (xi) size selecting one or more uncut target polynucleotides from said mixture of polynucleotides obtained in step (x).

The term "target polynucleotide" as used herein relates to any nucleic acid molecule of interest, which is amenable to molecular analysis. Preferably, the target polynucleotide is a DNA or cDNA molecule. The target polynucleotide is, in typical embodiments, derived from a test sample of a subject, or, in specific embodiments, a group of subjects. The term "target sequence" as used herein relates to a nucleic acid derived from the transcriptome of a subject or, in specific embodiments, a group of subjects. The target sequence may accordingly be provided as mRNA or, preferably, cDNA molecule. In specific embodiments of the present invention the target polynucleotides or the target sequences represent a gene, one or more exons of a gene, and/or an open reading frame or a sub-portion thereof. In further embodiments, the target polynucleotides or the target sequences may also be a panel of different genes, a panel of one or more exons of different genes, and/or a panel of open reading frames or sub-portions thereof. Further preferred are combinations of the before mentioned elements. The form and content of the target polynucleotide is typically reflected by the content and sequence of the target sequence derived from the transcriptome of a subject, which, in turn, is reflected by the sequence and form of the catcher oligonucleotides as used in the methods of the present invention.

In a first step of the method of the present invention a population of mRNA molecules is purified from a sample obtained from a subject.

The term "sample" or "test sample" as used herein relates to any biological material obtained via suitable methods known to the person skilled in the art from a subject. The sample used in the context of the present invention should preferably be collected in a clinically acceptable manner, more preferably in a way that nucleic acids, in particular RNA, more preferably mRNA molecules, are preserved. The biological samples may include body tissues and/or fluids, such as blood, or blood components like serum or plasma, sweat, sputum or saliva, semen and urine, as well as feces or stool samples. Furthermore, the biological sample may contain a cell extract derived from or a cell population including an epithelial cell, preferably a neoplastic epithelial cell or an epithelial cell derived from tissue suspected to be neoplastic. Particularly preferred are samples of cancerous tissue or comprising cancer cells. Also preferred are other disease-relevant tissue samples or other biological samples. Particularly preferred are liquid biopsy samples. Alternatively, the biological sample may be derived from animal sources. In certain embodiments cells may be used as primary sources for polynucleotides. In certain embodiments samples, in particular after initial processing, may be pooled. The present invention preferably envisages the use of non-pooled samples. In a specific embodiment of the present invention the content of a biological sample may also be submitted to a specific pre-enrichment step. For instance, a sample may be contacted with ligands specific for the cell membrane or organelles of certain cell types, functionalized for example with magnetic particles. The material concentrated by the magnetic particles may subsequently be used for the preparation of polynucleotides. In further embodiments of the invention, biopsy or resections samples may be obtained and/or used. Such samples may comprise cells or cell lysates. Furthermore, cells, e.g. tumor cells, may be enriched via filtration processes of fluid or liquid samples, e.g. blood, urine, sweat etc. Such filtration processes may also be combined with pre-enrichment steps based on ligand specific interactions as described herein above.

The term "purifying" or "purification" as used herein relates to the preparation and cleanup of nucleic acids, preferably in a manner which prevents degradation of the nucleic acids such as DNA or RNA. In a further step the cells may, for example, be cleaned from body tissues and fluids if necessary, and then further processed to obtain polynucleotides. The purification may be performed according to any suitable method known to the skilled person. Such methods may include the employment of nucleic acid extraction and washing protocols, the use of column based purification protocols, or, preferably, the employment of protocols based on magnetic beads. The protocols may either start with pretreated samples, or with crude samples. In preferred embodiments the purification is adapted to the preparation of RNA, in particular mRNA. Alternatively, the purification may be adapted to the preparation of DNA.

Accordingly, "polynucleotides obtained from a test sample" as used herein are typically polynucleotides which have been prepared from the test sample, e.g. DNA and/or RNA molecules, preferably DNA molecules. In addition, in certain embodiments, the polynucleotides may further have been purified, e.g. according to protocols mentioned above.

In a further step of the method of the present invention cDNA is prepared from the mRNA molecules previously obtained and purified. The preparation of cDNA is performed according to any suitable method or protocol known to the skilled person. Typically, a reverse transcription approach making use of poly-T and random oligonucleotides may be employed. For example, a poly-T oligonucleotide may be used which is complementary to the 3'-poly-A tail of mRNA molecules. The poly-T oligonucleotide may have any suitable length, e.g. 6 to 15 nucleotides.

As counter-oligonucleotide for the 3' region of the mRNA molecule, for example, a random oligonucleotide may be used. Such random oligonucleotides may comprise between 5 and 15 nucleotides with a random base sequence, i.e. without predefined sequence. The random base sequence typically covers all sequence possibilities in the covered stretch, including mono-nucleotide stretches such as poly-T, poly-A, poly-G, poly-C. Accordingly, the oligonucleotide is used as a group of different polynucleotides to cover all possibilities. The number of different oligonucleotides used for the annealing step depends on the size of the sequence covered with longer sequences necessitating more different oligonucleotides to cover all possible sequence variations than shorter sequences. It is preferred that the random oligonucleotide comprises 6, 7, 8, 9, or 10 nucleotides, i.e. that the random oligonucleotide is a hexamer, heptamer, octamer, nonamer or decamer. It is particularly preferred to use 6 nucleotides (hexamers).

In further specific embodiments, oligonucleotides used for the preparation of cDNA may be sequence-specific, or a combination of poly-T oligonucleotides and sequence-specific, i.e. non-random, oligonucleotides may be used. The employment of sequence-specific oligonucleotides allows for a reduction of the complexity of the group of obtained cDNAs to a predefined group. The sequence specific oligonucleotides may advantageously be associated with, i.e. used for copying, a specific gene, or a panel of genes, preferably a gene or part thereof or a panel of genes as defined herein, or any target sequence as defined herein.

In specific embodiments a group of specific oligonucleotides may be used, wherein said group covers, i.e. allows for copying, a gene, an exon, an open reading frame or sub-portion thereof in a consecutive manner. The term "consecutive" means that the specific oligonucleotides, when used for copying or amplification, cover in form of the obtained product or amplificate the entire sequence of said gene, exon, open reading frame or sub-portion thereof without an overlap, or, in further embodiments, with an overlap of 2, 3, 4, 5, 6, 7, 8, 9, 10, 15, or 20 or more nucleotides between each tiling piece.

The reverse transcription may be performed with any suitable reverse transcriptase known to the skilled person. Examples of such suitable reverse transcriptases are reverse transcriptases which do not comprise an RNase H-activity. Specific examples include MMLV reverse transcriptase without RNase H activity or commercially available reverse transcriptases such as SuperScript, SuperScript II, SuperScript III, StrataScript etc.

The reverse transcriptase reaction is preferably performed with or in the presence of a suitable buffer. Such a buffer may, for example, comprise TrisHCL, e.g. in a concentration of 250 mM, KCl, e.g. in a concentration of 375 mM, MgCl₂, e.g. in a concentration of 15 mM and DTT, e.g. in a concentration of 0.1 M, preferably at a pH of 8.3. In addition, a suitable amount of dNTPs, e.g. dATP, dCTP, dGTP and dTTP has to be used, e.g. in a suitable concentration such as 10mM.

Subsequent to the preparation of cDNA, the cDNA molecules are amplified. The amplification may preferably be performed as polymerase chain reaction (PCR). Suitable methods and protocols would be known to the skilled person. Examples of suitable PCR methods or PCR based methods for amplification of nucleic acids, which are envisaged by the present invention, include basic PCR, hot-start PCR, long PCR, quantitative endpoint PCR, rapid amplified polymorphic DNA analysis, rapid amplification of cDNA ends, differential display PCR, and high fidelity PCR. The amplification may preferably be performed with suitable polymerases, e.g. a Pfu DNA polymerase, or other thermostable DNA polymerase with high fidelity and the corresponding system. Further details would be known to the skilled person or can be derived from suitable literature sources such as: PCR, Methods and Protocols, 2017, Lucilia Domingues, Springer, New York, McClelland and Welsh, 1994, PCR Methods Appl., 4: S59-65. Alternative amplification methods also envisaged in the context of the present invention include NASBA, loop-mediated isothermal amplification with Bst-DNA-polymerase, isothermal amplification with Phi29 DNA polymerase, recombinase-polymerase amplification and helicase dependent isothermal amplification.

The amplification according to the present invention is typically performed on one or more target sequences as defined herein, e.g. a specific gene, or a panel of genes etc., preferably a gene or part thereof or a panel of genes as defined herein, or such that one or more such target sequences are comprised. The amplification of target sequences may accordingly be performed with sequence specific oligonucleotides, e.g. complementary to regions or segments of said target sequences. The amplification yields a pool of double stranded DNA molecules. The number of obtained molecule and the complexity of the pool can be adjusted via parameters of the PCR method, e.g. the number of PCR cycles, the hybridization temperature for the oligonucleotide annealing, buffer composition, the lengths of the oligonucleotide, temperature profiles and changes during the PCR etc.

In a further, specific embodiment of the present invention, the amplified target cDNA molecules may be fractionated, e.g. according to lengths, sequence, affiliation to a gene, panel of genes, gene family etc. The amplified cDNA molecules may either be used in a pooled manner or in a fractionated manner as described herein.

In a subsequent step of the method of obtaining an enriched personalized population of a target polynucleotide according to the present invention, the amplified DNA molecules are fragmented. The fragmentation may be performed according to any suitable method known to the skilled person. For example, the fragmentation may be achieved by restriction digest or any suitable shearing protocol, e.g. adaptive focused acoustic shearing (AFA) or Covaris shearing, use of nebulization forces, sonication, point-sink shearing or the use of a French press shearing procedure. It is preferred to use acoustic shearing, in particular the Covaris shearing. It is further preferred that the size of the polynucleotides obtained is similar or within a predefined range. The range of the obtained fragments may be between about 20 to 100 bp, more preferably between about 20 and 50 bp. In a particularly preferred embodiment, the resulting size of the fragmented DNA molecules is between about 20 to 30 bp.

The fragmented DNA molecules may be obtained as blunt end or sticky end fragments. Should the fragments be provided with sticky ends, they need to undergo a blunting reaction in order to prepared for a subsequent step of connecting with a tag. The blunting activity may, for example, be an end-repairing step. The end-repairing step may be performed by with any suitable end-repairing enzymatic activity, e.g. DNA polymerase I, preferably the Klenow fragment thereof, T4 DNA polymerase or T4 polynucleotide kinase. It is preferred that the end-repairing is performed with T4 DNA polymerase, T4 PNK and Klenow at 20 °C.

In a subsequent step, the fragments of step (iv), preferably further modified via end-repairing if necessary, as mentioned above, are connected to a tag which is capable of binding to a cognate interactor. This step yields a pool of tagged catcher oligonucleotides. The term "tag" as used herein relates to a molecule which is capable of binding to a cognate interactor and thereby be pulled out of a liquid solution or mixture of molecules. Examples of suitable tags and interactors are a biotin tag, e.g. DNA fragments obtained with steps described above, yielding "catcher oligonucleotides" and a streptavidin interactor provided on a suitable surface, e.g. of a reaction vessel, or on a bead etc. Also envisaged are derivatives of biotin and streptavidin as known to the skilled person. Further examples include a magnetic bead being bound to the DNA fragments obtained with steps described above, and a magnetic separator attracting said beads, e.g. attached at a surface. Also envisaged are embodiments in which the DNA fragments obtained with steps described above are immobilized at a surface or solid phase. Such an immobilization may, for example, be in the form of a column. The solid phase may have any suitable form or structure, e.g. be composed of sepharose. This allows to pass or run potentially hybridizing molecules through or over said surface or solid phase and to thereby bind them to the catcher oligonucleotide and thus remove them from the pool. This activity may, in certain embodiments, be repeated one or several times. The interactor-tag binding may thus advantageously be used to pull said catcher oligonucleotides together with any bound or associated binding partner comprising a complementary target sequence from a mixture of liquid solution.

Typically, the catcher oligonucleotide hence corresponds to a portion of a polynucleotide, which should be enriched or should be comprised in an enriched personalized population of target polynucleotides. For example, the catcher oligonucleotide is complementary to a target polynucleotide or a polynucleotide comprising a target sequence as defined herein, e.g. covering a gene, one or more exons of a gene, and/or an open reading frame or a sub-portion thereof, or, in further embodiments, covering a panel of different genes, a panel of one or more exons of different genes, and/or a panel of open reading frames or sub-portions thereof, as well as combinations of the before mentioned elements. It is preferred that panels of genes etc. are covered by groups of catcher oligonucleotides. The specificity, subject-dependency and hence personalization of said genes or panel of genes etc. is achieved by using subject sample derived RNA molecules.

The term "connected" as used herein refers to any suitable step which links the DNA fragment with the tag as described above. The connection to the tag is performed at both strands of a double stranded amplified cDNA fragment. The connection may, preferably, be performed at the 3' terminus of each strand. Such linking procedure may, for example, comprises an end-tailing with an activated tag, e.g. biotin. Preferably, an A-tailing of blunted cDNA fragments is performed, wherein the A-tailing step includes, in a preferred embodiment, the use of biotinyl-dATP. The A-tailing activity may be performed by any suitable A-tailing enzymatic activity such as Taq polymerase or Klenow fragment. The A-tailing is preferably carried out with Taq DNA polymerase at 65°C. Further details can be derived from suitable literature sources such as Nucleic Acids Research, 2010, 38, 13, e137.

Alternatively, the connection reaction may be based on a ligation reaction with a tag, e.g. biotin, or a linkage to tag, e.g. biotin, via click chemistry.

The ligation may be a chemical or an enzymatic ligation. The enzymatic ligation is preferred. A chemical ligation typically requires the presence of condensing reagents. An example of a chemical ligation envisaged by the present invention makes use of electrophilic phosphorothioester groups. Further examples include the use of cyanogen bromide as a condensing agent. The enzymatic ligation may be performed with any suitable enzymatic ligase known to the skilled person. Examples of suitable ligases include T4 DNA ligase, E. coli DNA ligase, T3 DNA ligase and T7 DNA ligase. Alternatively, ligases such as Taq DNA ligase, Tma DNA ligase, 9°N DNA ligase, T4 Polymerase 1, T4 Polymerase 2, or Thermostable 5' App DNA/RNA ligase may be used.

The connection of the tag to the cDNA fragment may be performed via click chemistry. The term "click chemistry" relates to a reaction between an azide and an alkyne yielding a covalent 1,5-disubstituted 1,2,3-triazole product and is essentially based on Cu catalysis. Typically, the catalyst may be introduced as Cu-TBTA complex. It is preferred that the linkage between the cDNA fragment and the tag, e.g. biotin, is implemented via the introduction of an alkynyl residue into the DNA molecule. Tis is typically performed via termination reaction or nick translation reactions using alkynyl triphosphate. Alternatively, the alkynyl may also be introduced during PCR. This reaction yields an alkyne-modified DNA which can react with a cognate azide activated ester, preferably an azide activated tag, more preferably an azide activated biotin.

The tagged catcher oligonucleotide is subsequently provided as single stranded molecule. Accordingly, the double strand molecules, comprising tags at both strands, are melted to obtain single strands. In specific embodiments, the single stranded nature of the catcher oligonucleotides is maintained or improved by adding suitable buffers or using suitable reaction parameters.

In a further step of the method of obtaining an enriched personalized population of a target polynucleotide according to the present invention, a pool of starting oligonucleotides for the preparation of synthetic single guide RNA (sgRNA) for an sgRNA-guided nucleic acid-binding protein is provided.

The corresponding part of the method is, in general, based on the employment of the CRISPR/Cas system. The term "CRISPR/Cas system" as used herein relates to a biochemical method to specifically cut and modify nucleic acids, also known as genome editing. For example, genes in a genome can generally be inserted, removed or switched off with the CRISPR/Cas system, nucleotides in a gene or nucleic acid molecule can also be changed. The effect of the concept and activity steps of the CRISPR/Cas system has various similarities to that of RNA interference, since short RNA fragments of about 18 to 20 nucleotides mediate the binding to the target in both bacterial defense mechanisms. In the CRSIPR/Cas system typically RNA-guided nucleic acid-binding proteins, such as Cas proteins, bind certain RNA sequences as ribonucleoproteins. For example, a Cas endonuclease (e.g. Cas9, Cas5, Csn1 or Csx12, or derivatives thereof) can bind to certain RNA sequences termed crRNA repeats and cut DNA in the immediate vicinity of these sequences. Without wishing to be bound by theory, it is believed that the crRNA repeat sequence forms a secondary RNA structure and is then bound by the nucleic acid-binding protein (e.g. Cas) which alters its protein folding allowing the target DNA to be bound by the RNA. Furthermore, the presence of a PAM motif, i.e. a protospacer adjacent motif, in the target DNA is necessary to activate the nucleic acid-binding protein (e.g. Cas). The DNA is typically cut three nucleotides before the PAM motif. The crRNA repeat sequence is typically followed by a sequence binding to the target DNA, i.e. a crRNA spacer; both sequences, i.e. the crRNA repeat motif and the target binding segment are usually labelled as "crRNA". This second part of the crRNA (target binding segment) is a crRNA-spacer sequence having the function of a variable adapter. It is complementary to the target DNA and binds to said target DNA. An additional RNA, a tracrRNA, or trans-acting CRISPR RNA, is also required. tracrRNA is partially complementary to crRNA, so that they bind to each other. tracrRNA typically binds to a precursor crRNA, forms an RNA double helix and is converted into the active form by RNase III. These properties allow for a binding to the DNA and a cutting via the endonuclease function of the nucleic acid-binding protein (e.g. Cas) near the binding site.

The term "starting oligonucleotide" as used herein relates to a short nucleic acid molecule or nucleic acid oligomer. Its lengths may vary according to the specific application, targeting approach, genetic background of involved organisms etc. Typically, the length of the starting oligonucleotide is between about 40 and 250 nucleotides, e.g. 40, 45, 50, 55, 60, 65, 100, 150, 200 or 250 nucleotides or any value in between the mentioned values. It is preferred that the length of the oligonucleotide is 55 nucleotides. It is preferred that the starting oligonucleotide is a single strand DNA molecule. Also envisaged, in specific alternative embodiments, are RNA, PNA, CNA, HNA, LNA or ANA molecules or mixtures thereof as starting oligonucleotides. The term "PNA" as used herein relates to a peptide nucleic acid, i.e. an artificially synthesized polymer similar to DNA or RNA. The PNA backbone is typically composed of repeating N-(2-aminoethyl)-glycine units linked by peptide bonds. The various purine and pyrimidine bases are linked to the backbone by methylene carbonyl bonds. The term "CNA" as used herein relates to a cyclopentane nucleic acid, i.e. a nucleic acid molecule comprising for example 2'-deoxycarbaguanosine. The term "HNA" relates to hexitol nucleic acids, i.e. DNA analogues which are built up from standard nucleobases and a phosphorylated 1, 5-anhydrohexitol backbone. The term "LNA" as used herein relates to locked nucleic acids. Typically, a locked nucleic acid is a modified and thus inaccessible RNA nucleotide. The ribose moiety of an LNA nucleotide may be modified with an extra bridge connecting the 2' and 4' carbons. Such a bridge locks the ribose in a 3'-endo structural conformation. The locked ribose conformation enhances base stacking and backbone pre-organization. This is assumed to increase the thermal stability, i.e. melting temperature of the oligonucleotide. The term "ANA" as used herein relates to arabinoic nucleic acids or derivatives thereof. A preferred ANA derivative in the context of the present invention is a 2'-deoxy-2'-fluoro-beta-D-arabinonucleoside (2'F-ANA).

The oligonucleotides are typically provided in a liquid, e.g. aqueous, solution. The solution may comprise or be composed of suitable buffers such as a hybridization buffer, e.g. comprising SSC, NaCl, sodium phosphate, SDS, TE and/or MgCl₂.

The "pool of starting oligonucleotides for the preparation of synthetic single guide RNA (sgRNA)" as used herein relates to a group of oligonucleotides which provide features necessary to prepare a pool of synthetic single guide RNAs (sgRNA). In this context the term "synthetic single guide RNA (sgRNA)" or "single guide RNA (sgRNA)" as used herein relates to an artificial or synthetic combination of a crRNA and a tracrRNA sequence of the CRISPR/Cas system as described above. Typically, the sgRNA comprises a sequence segment which can be used to guide a DNA binding protein towards the binding site. As described in Jinek et al., 2012, Science, 337, 816-821 crRNA and tracrRNA can be combined into a functional species (sgRNA) which fulfills both activities (crRNA and tracrRNA) as mentioned above. For example, nucleotides 1-42 of crRNA-sp2, nucleotides 1-36 of crRNA-sp2 or nucleotides 1-32 of crRNA-sp2 may be combined with nucleotides 4-89 of tracrRNA. Further options for obtaining an sgRNA can be derived from Nowak et al., 2016, Nucleic Acids Research, 44, 20, 9555-9564. For example, an sgRNA may be provided which comprises different forms of an upper stem structure, or in which the spacer sequence is differentially truncated from a canonical 20 nucleotides to 14 or 15 nucleotides. Further envisaged variants include those in which a putative RNAP III terminator sequence is removed from the lower stem. Also envisaged is a variant, in which the upper stem is extended to increase sgRNA stability and enhance its assembly with an sgRNA-guided nucleic acid-binding protein, e.g. Cas protein. According to further embodiments of the present invention, the sequence and form of the sgRNA may vary in accordance with the form or identity of the sgRNA-guided nucleic acid-binding protein, e.g. Cas protein. Accordingly, depending on the origin of said sgRNA-guided nucleic acid-binding protein, a different combination of sequence elements may be used. The present invention further envisages any future development in this context and includes any modification or improvement of the sgRNA-nucleic acid-binding protein interaction surpassing the information derivable from Jinke et al., 2012 or Nowak et al.,2016. In specific embodiments, the sgRNA to be used may have the sequence of any one of SEQ ID NO: 1 to 3. Particularly preferred is the use of a Streptococcus pyogenes sgRNA, e.g. as used in commercially available kits such as EnGen sgRNA synthesis Kit provided by New England Biolabs Inc. Also envisaged are similar sgRNA forms from other commercial suppliers, or individually prepared sgRNAs. Such sgRNAs may be derived from the sequence of SEQ ID NO: 1 if used with a cognate nucleic acid-binding protein form S. pyogenes. Alternatively, the sgRNA may be derived from the sequence of SEQ ID NO: 2 if used with a cognate nucleic acid-binding protein form Staphylococcus aureus. In a further alternative, the sgRNA may be derived from the sequence of SEQ ID NO: 3 if used with a cognate nucleic acid-binding protein form Streptococcus thermophilus.

The features necessary to prepare a synthetic single guide RNA (sgRNA), in general, comprise all elements which are necessary to generate an sgRNA molecule suitable for employment in a CRISPR/Cas system as described herein above. Accordingly, these features include the presence of a promoter segment; the presence of a random segment which is or comprises a potentially complementary sequence for a catcher oligonucleotide as described herein, thus serving as complementary sequence for a potential binding or hybridization interactor having a matching sequence; and the presence of a binding element which is complementary to at least a portion of a scaffold sequence for interaction with the sgRNA-guided nucleic acid-binding protein. The mentioned features may be provided in any suitable order. It is preferred that the order is, from 5' to 3': (i) promoter segment, (ii) random segment and (iii) biding element for scaffold sequence. The members of said group of oligonucleotides differ by the sequence of the random segment.

The "promoter segment" as used herein relates to any suitable promoter structure, which is capable of initiating RNA transcription. It is preferred that the promoter is a promoter which operates under in vitro conditions. In further embodiments, the promoter may be a constitute promoter or a regulable promoter. An example of a suitable promoter is the T7 RNA polymerase promoter. In alternative embodiments, the promoter may be an U6 RNA polymerase III promoter, a type III RNA polymerase III promoter H1, or a Cytomegalovirus promoter (CMV), preferably a minimal CMV promoter. The promoter segment may be accompanied or additionally comprise further elements such as spacer elements, guiding elements etc. For example, upstream of a spacer sequence of the promoter the segment may preferably comprise 1 or 2 guanine residues. Further details for the promoter segment may be derived from suitable literature sources such as Milligan et al., 1987, Nucleic Acids Research, 15, 21, 8783-8798 or Nowak et al., 2016, Nucleic Acids Research, 44, 20, 9555-9564.

The "random segment" as used herein relates to a nucleic acid stretch comprising random base sequences, which typically cover all sequence possibilities in the covered stretch, including mono-nucleotide stretches such as poly-T, poly-A, poly-G, poly-C. In certain embodiments a majority or a certain amount of possible nucleotide combinations or sequences may be represented by the random segments, e.g. 99%, 95%, 90%, 85%, 80%, 75%, 70% or less or any value in between the mentioned values. In preferred embodiments the random segment comprises between about 10 to 30 random nucleotides, e.g. 10, 11, 12, 13, 14, 15, 16, 17, 18, 19, 20, 21, 22, 23, 24, 25, 26, 27, 28, 29 or 30 nucleotides. It is particularly preferred that the random segment comprises about 20 nucleotides.

The "pool" of starting oligonucleotides may have any suitable size. Typically, the size of the pool is dependent on the length of the random segment such that a longer sequence implies a bigger pool of oligonucleotides with longer segments necessitating more different oligonucleotides to cover all possible sequence variations than shorter segments. The pool of oligonucleotides may accordingly comprise segments with a random base sequence, i.e. without predefined sequence thus comprising all possible nucleotide combinations or sequences in said segment.

In specific embodiments certain random segments may be overrepresented, while other may be underrepresented. This over- or underrepresentation may be controlled on purpose, e.g. according to necessities, known or expected sequence compositions in a sample or the addition of separation or elimination techniques used during sample preparation, e.g. length exclusions etc. For example, it may be advantageous to provide a higher representation of sequences for nucleic acid species which are present more frequently in a test sample such as rDNA sequences, repetitive sequences etc.

In further embodiments, the pool of oligonucleotides may comprise a one time or several times representation of a single specific nucleotide combination or sequence. For example, each possible nucleotide combination or sequence in the random segment as defined above may be represented in the pool of oligonucleotides 1 time, 2 times, 3 times, 4 times, 5 times, 10 times, 50 times, 100 times, 1000 times or more often.

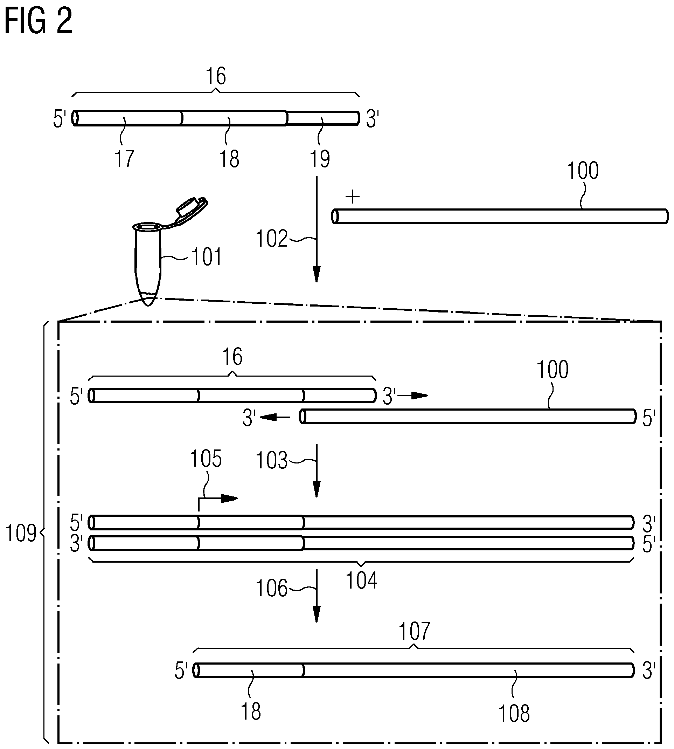

The term "binding element which is complementary to at least a portion of a scaffold sequence for interaction with the sgRNA-guided nucleic acid-binding protein" relates to a nucleic acid segment, which comprises a sequence being complementary to an oligonucleotide comprising crRNA and tracrRNA functionalities as combined in sgRNA, preferably as described herein above, comprising a crRNA repeat motif and an RNA double helix forming element. The term "scaffold sequence" as used herein relates to said structural motifs which are typically required for the binding of and interaction with the sgRNA-guided nucleic acid-binding protein, e.g. Cas protein, as defined above. By providing said scaffold functionality in separate oligonucleotides, a hybridization step between the pool of starting oligonucleotides, preferably a reduced pool of starting oligonucleotides according to the invention, and said scaffold sequence, followed by DNA extension leads to a double stranded template molecule comprising a promoter segment, a random segment as potentially complementary sequence for the catcher oligonucleotide and said sgRNA scaffold functionality in one entity. The process of obtaining said double stranded template molecule comprising a promoter segment, a random segment as potentially complementary sequence for the catcher oligonucleotide and said sgRNA scaffold functionality in one entity, as well as the subsequent step of generating an sgRNA molecule on the basis of said template molecule is schematically depicted in Fig. 2.

The term "complementary" as used herein refers to the presence of matching base pairs in opposite nucleic acid strands. For example, to a nucleotide or base A in a sense strand a complementary or antisense strand binds with a nucleotide or base T, or vice versa; likewise to a nucleotide or base G in a sense strand the complementary or antisense strand binds with a nucleotide or base C, or vice versa. This scheme of complete or perfect complementarity may, in certain embodiments of the invention, be modified by the possibility of the presence of single or multiple non-complementary bases or stretches of nucleotides within the sense and/or antisense strand(s). Thus, to fall within the notion of a pair of sense and antisense strands, both strands may be completely complementary or may be only partially complementary, e.g. show a complementarity of about 80%, 85%, 90%, 91%, 92%, 93%, 94%, 95%, 96%, 97%, 98%, 99%, 99.5%, or 100% between all nucleotides of both strands or between all nucleotides in specific segments as defined herein. Non-complementary bases may comprise one of the nucleotides A, T, G, C, i.e. show a mismatch e.g. between A and G, or T and C, or may comprise any modified nucleoside bases including, for example, modified bases as described in WIPO Standard ST.25. Furthermore, the present invention also envisages complementarity between non-identical nucleic acid molecules, e.g. between a DNA strand and a RNA strand, a DNA strand and a PNA strand, a DNA strand and a CNA strand, etc. It is preferred that the complementarity between strands or segments as defined herein is a complete or 100% complementarity.

The term "complementary to at least a portion of a scaffold sequence" as used herein means that the binding segment has a complementary overlap with said oligonucleotide comprising the scaffold sequence. The overlap may, for example, be an overlap of 5, 7, 10, 12, 15, 18, 20, 22, 25, 28 or 30 nucleotides, or any value in between the mentioned values. Also envisaged are longer overlaps. Preferred are short overlaps in the range of 5 to 20 nucleotides. The length of the overlap may further be adjusted in view of hybridization efficiency. The overlap typically is at the 3' end of the starting oligonucleotide and at the 5' end of the oligonucleotide comprising the scaffold sequence. Within said overlap the matching or complementarity between the complementary bases is preferably 100%. In alternative embodiments, the matching is less than 100%, e.g. 99%, 95%, 90%, 85% or less than 85%.

The term "potentially complementary sequence" as used in the context to the random segment relates to a sequence of the random segment which can be, with a certain likelihood depending on the size, nucleotide combination etc. be complementary to one or more catcher oligonucleotides as defined herein above, which are derived from cDNA/mRNA molecules of a subject. Accordingly, the random segment may comprise sequences which are complementary to said catcher oligonucleotides and sequences which are not complementary to said catcher oligonucleotides. The gist of the invention is to identify or catch those starting oligonucleotides for the preparation of synthetic single guide RNA (sgRNA) which are indeed complementary to the personalized catcher oligonucleotides, allowing for a tailored removal of these sequences from the pool of starting oligonucleotides.

In case of an at least partial complementarity between catcher oligonucleotide and starting oligonucleotide the hybridization preferably takes place between said catcher oligonucleotide and at least a portion of the potentially complementary sequence provided in the starting oligonucleotide as defined herein. The binding between these molecules is capable of efficiently removing a starting oligonucleotide comprising said complementary sequence from the pool of starting oligonucleotides.

Preferably, the catcher oligonucleotide comprises between about 20 to 30 nucleotides, e.g. 20, 21, 22, 23, 24, 25, 26, 27, 28, 29 or 30 nucleotides. The catcher oligonucleotide may further comprise additional elements such as spacer elements, barcoding sequences etc. These elements may be added to the catcher oligonucleotide before or after the connection to a tag as described above.

In a subsequent step said pool of starting oligonucleotides and said catcher oligonucleotides are hybridized. The hybridization typically takes place in a liquid solution, e.g. an aqueous solution comprising a suitable buffer as defined. The hybridization may be performed in accordance with any suitable temperature, ion concentration and/or pH parameter known to the skilled person. For example, the hybridization may be performed at a temperature and/or pH and/or ionic concentration in the solution at which a complementary base-pairing between most, preferably all complementary bases in the starting oligonucleotide and the catcher oligonucleotide takes place. Unspecific binding or hybridization reactions may, for example, be avoided by setting the temperature to a value which only allows for a complete, i.e. 100 % complementary binding. In alternative embodiments, the temperature may be set to a value, which allows for a complementary binding of about 99%, 98%, 95%, 90%, 85% or 80% of complementary bases.

In a further step of the method of obtaining an enriched personalized population of a target polynucleotide of the present invention complexes of starting oligonucleotides and catcher oligonucleotides are removed from the pool of starting oligonucleotides. The removal is preferably initiated by binding said tag to a cognate interactor, preferably located on a bead or a suitable surface. For example, by introducing beads comprising streptavidin molecules a biotin tag may be associated to said tag. Furthermore, hybridized starting oligonucleotides, i.e. those oligonucleotides which comprise a sequence matching the sequence of the catcher oligonucleotide are indirectly also bound to the bead. Subsequently, the beads with the associated nucleic acids can be removed from the solution. For example, the bead may be a magnetic bead which can be removed via magnetic force. Also envisaged are different removal options such as centrifugation or filtration. Alternatively, the removal may be implemented by magnetic beads - magnetic force interaction. In such a scenario the catcher oligonucleotide is linked to a magnetic bead. After hybridization with a matching starting oligonucleotide a magnetic force is applied and the complex between catcher oligonucleotide and starting oligonucleotide can be removed from the solution, e.g. towards a magnetic zone.

After having performed the above outlined step the pool of starting oligonucleotides is reduced, i.e. starting oligonucleotides complementary to the sequence of the catcher oligonucleotide are no longer present in the pool or their presence has been reduced. The term "reduced presence" as used herein means that the number of starting oligonucleotides complementary to the sequence of the catcher oligonucleotide has been reduced 5-fold, 10-fold, 20-fold, 30-fold, 40-fold, 50-fold, 100-fold.

In a specific embodiment the pervious steps (vii) and (viii), i.e. the step of hybridizing the pool of starting oligonucleotides and said tagged catcher oligonucleotide(s), and the step of removing complexes of starting oligonucleotides and tagged catcher oligonucleotides as defined above, may be repeated one or several times. For example, these steps may be repeated 1, 2, 3, 4, 5 or more times. The repetition may, in certain embodiments, be connected with an amplification step, e.g. via PCR, of the reduced pool of starting oligonucleotides. Accordingly, a suitable primer binding site on each starting oligonucleotide may be present and be used for the amplification. The amplification may preferably be performed with a Pfu DNA polymerase, or other thermostable DNA polymerase with high fidelity and the corresponding system. Each repetition may be combined with a washing step and or a quality control step. For example, the presence of certain elements in the reduced pool may be determined with real-time PCR. In specific embodiments, each repetition is performed with a new catcher oligonucleotide or new group of catcher oligonucleotides. In further embodiments, each repetition may be performed with a different catcher oligonucleotide or a different group of catcher oligonucleotides, e.g. with one or more fractions of the cDNA molecules as described herein above. The difference may, for example, be a different portion of the overall target sequence, e.g. an adjacent sequence portion of a gene or genomic sequence if said gene is covered by several adjacent or consecutive or partially overlapping catcher oligonucleotides.

In a further step a pool of sgRNAs with said reduced pool of starting oligonucleotides obtained in step (viii) is generated. This step may typically be performed as depicted in Fig. 2. Typically, an single stranded oligonucleotide, preferably a DNA molecule, comprising crRNA and tracrRNA functionalities as combined in sgRNA, preferably as described herein above, comprising a crRNA repeat motif and an RNA double helix forming element is hybridized to a starting oligonucleotide obtained in step (viii) via the binding element present as defined herein above, which is present in said starting oligonucleotide. Subsequently, single stranded portions of the hybrid complex may be filled via DNA extension reactions. This reaction is preferably performed with a DNA polymerase, e.g. a T4 DNA polymerase, or Klenow enzyme.

The resulting double stranded template molecule comprising a promoter segment, a random segment as which is irrelevant for the binding to the catcher oligonucleotide, i.e. does not show complementarity to the sequence of the catcher oligonucleotide, and said sgRNA scaffold functionality in one entity may, in certain embodiments, be amplified, e.g. via PCR. Subsequently, the template can be transcribed into an RNA molecule via the promoter segment as defined herein above, yielding an sgRNA which can be used for CRISPR/Cas activities.

In a specific embodiment, the pool of sgRNAs is obtained according to a commercial protocol and on the basis of a commercial kit as, for example, provided by New England Biolabs such as the EnGen sgRNA synthesis Kit. Also envisaged are similar sgRNA forms form other commercial suppliers.

Accordingly, in a further aspect, the present invention relates to a method of obtaining a pool of personalized target-irrelevant synthetic single guide RNAs (sgRNAs) for a sgRNA-guided nucleic acid-binding protein. This method comprises essentially the steps (i) to (ix) as defined herein. In an alternative aspect the present invention envisages the provision of a reduced pool of starting oligonucleotides comprising essentially the steps (i) to (iv) as defined herein above.

In certain embodiments, the sgRNA obtained in step (ix) may be stored, modified and/or purified in order to allow for a suitable further usage. For example, potentially present 5' triphosphate residues may be removed, e.g. by employing alkaline phosphatase. A purification of the sgRNA may be performed according to any suitable protocol known to the skilled person, e.g. with a spin column to remove proteins, salts and nucleotides. Also envisaged is, in certain embodiments, a quality check of the sgRNA before further usage, e.g. via UV light absorbance at 260 nm.

In a further step of the method of obtaining an enriched personalized population of a target polynucleotide of the present invention a mixture of polynucleotides obtained from a test sample as defined herein is cleaved with an sgRNA-guided nucleic acid-binding protein using the pool of sgRNAs obtained in step (ix) as defined above.

The term "mixture of polynucleotides" as used herein relates to nucleic acids derived from a sample as mentioned above. The polynucleotide to be use in accordance with this step of the method according to the present invention is preferably a DNA molecule or a cDNA molecule. The DNA molecule may be a genomic DNA or a derivative thereof. Also envisaged is the use of DNA libraries.

In preferred embodiments, the mixture of polynucleotides obtained from a test sample, especially from a liquid biopsy sample, comprises genomic DNA and/or cDNA molecules fragments called circulating cell free DNA (ccfDNA). The size of these DNA species is typically between 70-300 base pairs. The ccfDNA typically comprises degraded DNA fragments released to the blood plasma. ccfDNA can be used to describe various forms of DNA freely circulating the bloodstream, including circulating tumor DNA (ctDNA) and cell-free fetal DNA (cffDNA). Elevated levels of cfDNA are observed in cancer, especially in advanced disease. There is evidence that cfDNA becomes increasingly frequent in circulation with the onset of age. ccfDNA has been shown to be a useful biomarker for a multitude of ailments other than cancer and fetal medicine. This includes but is not limited to trauma, sepsis, aseptic inflammation, myocardial infarction, stroke, transplantation, diabetes, and sickle cell disease. ccfDNA is mostly a double-stranded extracellular molecule of DNA.

In preferred embodiments, mixture of polynucleotides obtained from a test sample comprises genomic DNA and/or cDNA molecules whose size is optionally adjusted to a predefined value. For example, a mixture of polynucleotides may comprise genomic DNA or cDNA molecules which are sheared or fragmented as defined above. In an optional embodiment, the size of the polynucleotides obtained may be adjusted to a predefined range. Exemplary ranges are about 2 kb to 2.5 kb, 2.5 kb to 3 kb, or 3 kb to 3.5 kb etc., 5 kb to 6 kb, 10 kb to 12 kb etc. The size of the polynucleotides, as well as any optional adjustment, may depend on the target sequence length as mentioned herein.

The cleavage with an sgRNA-guided nucleic acid-binding protein, e.g. a nuclease such as Cas, using the pool of sgRNAs obtained in step (ix) is performed according to the CRSIPR/Cas method as described above. Typically, the mixture of polynucleotides as defined above is added to a reaction solution comprising the sgRNA as mentioned above in a suitable concentration, a suitable reaction buffer and an sgRNA-guided nucleic acid-binding protein in a suitable concentration. The reaction may be incubated at a suitable temperature. Subsequently, the reaction may, in certain embodiments, be stopped by the addition of a proteinase, e.g. proteinase K or, preferably, by performing a heat denaturation step, e.g. at 65°C.

In a specific embodiment, the cleavage may also be performed according to a commercial protocol and on the basis of a commercial kit as, for example, provided by New England Biolabs such as the EnGen Cas9 NLS, S. pyogenes in vitro digestion kit. Also envisaged are similar digestion protocols form other commercial suppliers.

In a final step of the method of obtaining an enriched personalized population of a target polynucleotide of the present invention a size selection is performed which allows to separate uncut target polynucleotides from cleaved polynucleotides as obtained in step (x). Typically, due to the use of a random target sequence for the sgRNA preparation, polynucleotide molecules comprising a matching random sequence are cleaved with the CRSIPR/Cas system. Polynucleotide molecules which comprise a target sequence, which is not recognized by the sgRNAs in the pool of sgRNAs since these molecules have been removed via the hybridization with the catcher oligonucleotide as described above, i.e. target polynucleotides according to the present invention, will not be cleaved and accordingly have a larger size. The size selection can be performed with any suitable method. For example, an agarose gel- or polyacrylamide gel-based approach or a bead-based approach may be used. In preferred alternative embodiments magnetic beads may be used to remove short fragments.

Obtained target polynucleotides, may subsequently be purified, stored and/or used for additional or later activities.

In a specific embodiment of the present invention, one additional activity to be performed with said polynucleotides is the sequencing said obtained target polynucleotide. The term "sequencing" as used herein relates to any suitable sequencing methodology known to the skilled person. Preferably, a next-generation sequence (NGS) or second generation sequencing technique may be used, which is usually a massively parallel sequencing approach performed in a highly parallel fashion. The sequencing may, for example, be performed according to parallel sequencing approach on platforms such as Roche 454, GS FLX Titanium, Illumina, Life Technologies Ion Proton, Oxford Nanopore Technologies, Solexa, Solid or Helicos Biosciences Heliscope systems. The sequencing may, in certain embodiments, also include an additional preparation of polynucleotides, the sequencing, as well as subsequent imaging and initial data analysis steps.

Preparation steps may, for example, include randomly breaking polynucleotides into smaller sizes and generating sequencing templates such as fragment templates. Spatially separated templates can, for example, be attached or immobilized at solid surfaces which allows for a sequencing reaction to be performed simultaneously. In typical examples, a library of nucleic acid fragments is generated and adaptors containing universal priming sites are ligated to the end of the fragments. Subsequently, the fragments are denatured into single strands and captured by beads. After amplification a huge number of templates may be attached or immobilized in a polyacrylamide gel, or be chemically crosslinked to an amino-coated glass surface, or be deposited on individual titer plates. Alternatively, solid phase amplification may be employed. In this approach forward and reverse primers are typically attached to a solid support. The surface density of amplified fragments is defined by the ratio of the primers to the template on the support. This method may produce millions of spatially separated template clusters which can be hybridized to universal sequencing primers for massively parallel sequencing reactions. Further suitable options include multiple displacement amplification methods. Suitable sequencing methods include, but are not limited to, cyclic reversible termination (CRT) or sequencing by synthesis (SBS) by Illumina, sequencing by ligation (SBL), single-molecule addition (pyrosequencing) or real-time sequencing. Exemplary platforms using CRT methods are Illumina/Solexa and HelicoScope. Exemplary SBL platforms include the Life/APG/SOLiD support oligonucleotide ligation detection. An exemplary pyrosequencing platform is Roche/454. Exemplary real-time sequencing platforms include the Pacific Biosciences platform and the Life/Visi-Gen platform. Other sequencing methods to obtain massively parallel nucleic acid sequence data include nanopore sequencing, sequencing by hybridization, nano-transistor array based sequencing, scanning tunneling microscopy (STM) based sequencing, or nanowire-molecule sensor based sequencing. Further details with respect to the sequencing approach would be known to the skilled person, or can be derived from suitable literature sources such as Goodwin et al., 2016, Nature Reviews Genetics, 17, 333-351, van Dijk et al., 2014, Trends in Genetics, 9, 418-426 or Feng et al., 2015, Genomics Proteomics Bioinformatics, 13, 4-16.

In a further aspect the present invention relates to a target polynucleotide obtainable by the method of obtaining an enriched personalized population of a target polynucleotide as defined herein above. The target polynucleotide may accordingly be present in a mixture with non-target polynucleotides, e.g. in a size fractionable state. Accordingly, by separating the target polynucleotide from non-target polynucleotides as described herein a pure fraction of target polynucleotides may be obtained. Similarly, the target polynucleotide may be present in a separated form in gel such as an agarose gel or polyacrylamide gel and can thus be extracted therefrom with suitable methods known to the skilled person. The target polynucleotide obtained may be purified, stored or modified according to any suitable approach. The target polynucleotide may be provided in any suitable buffer or liquid, or it may be provided in dried or lyophilized form.

In a further aspect the present invention relates to a pool of target-irrelevant synthetic single guide RNAs (sgRNAs) for a sgRNA-guided nucleic acid-binding protein, e.g. Cas9, obtained according to a method of the present invention. The pool may accordingly be provided as RNA molecule. It is preferred that the RNA has been purified and/or cleaned. It may be provided in any suitable buffer or liquid, or may be provided in dried or lyophilized form. In an alternative embodiment, the pool of sgRNAs is provided as reduced pool of starting oligonucleotides in accordance with the present invention. In a further example, it may be provided as mixture of starting oligonucleotides and scaffold sequence containing oligonucleotides as described above.

In a further aspect the present invention relates to a kit comprising a pool of sgRNAs obtainable by the method of obtaining a pool of personalized target-irrelevant synthetic single guide RNAs (sgRNAs) for a sgRNA-guided nucleic acid-binding protein and a sgRNA-guided nucleic acid-binding protein. The kit is preferably for enriching a personalized population of a target polynucleotide. The features of the methods as defined herein above apply also to the kit of the present invention. The kit may, for example, comprise reagents and components as defined in one or more steps of the present methods. For example, the kit may comprise reagents or components for cleaving a mixture of polynucleotides obtained from a test sample with an sgRNA-guided nucleic acid-binding protein. In a different embodiment, the kit may comprise or may comprise in addition reagents or components for performing a size selection. The kit may, in general, comprise suitable buffer solutions, labels or washing liquids etc. Furthermore, the kit may comprise an amount of a known nucleic acid molecule or protein, which can be used for a calibration of the kit or as an internal control. Corresponding ingredients would be known to the skilled person.

Additionally, the kit may comprise an instruction leaflet and/or may provide information as to its usage etc.

Also envisaged is an apparatus performing the above mentioned method steps. The apparatus may, for example, be composed of different modules which can perform one or more steps of the method of the present invention. These modules may be combined in any suitable fashion, e.g. they may be present in a single place or be separated. Also envisaged is the performance of the method at different points in time and/or in different locations. Some steps of the method as defined herein may be followed by breaks or pauses, wherein the reagents or products etc. are suitably stored, e.g. in a freezer or a cooling device. In case these steps are performed in specific modules of an apparatus as defined herein, said modules may be used as storage vehicle. The modules may further be used to transport reaction products or reagents to a different location, e.g. a different laboratory etc.

In yet another aspect the present invention relates to the use of a pool of sgRNAs obtainable by the method of obtaining a pool of target-irrelevant synthetic single guide RNAs (sgRNAs) for a sgRNA-guided nucleic acid-binding protein for the removal of target-irrelevant polynucleotides from a mixture of polynucleotides in a sgRNA-guided nucleic acid-binding protein-based assay. The assay may comprise the step of cleaving a mixture of polynucleotides obtained from a test sample with an sgRNA-guided nucleic acid-binding protein wherein said nucleic acid-binding protein is guided towards the sequence to be cleaved by the sgRNA obtained in accordance with the methods of the present invention. The features of the methods as defined herein above apply also to the use or assay as mentioned above.

In preferred embodiments of the above mentioned methods, kits or uses the sgRNA-guided nucleic acid-binding protein is a DNA binding Cas protein. Examples of such DNA binding Cas proteins are Cas2, Cas3, Cas5, Csn1 or Csx12 or Cas9. Also envisaged are derivatives thereof or mutants. In particularly preferred embodiments, the sgRNA-guided nucleic acid-binding protein is derived from a family of Cas9 proteins or derivatives thereof. It is even more preferred that the sgRNA-guided nucleic acid-binding protein is Cas9 or a derivative thereof. The derivative is preferably a functional derivative which has a nuclease activity. The present invention further envisages the use of Cas9 derived from different bacterial sources. For example, the Cas9 protein may be derived from Streptococcus pyogenes, Staphylococcus aureus, or Streptococcus thermophiles. It is preferred that the Cas9 is a Streptococcus pyogenes Cas9 protein. Further details on the form and use of Cas proteins may be derived from suitable literature sources such as Jiang and Doudna, 2017, Annu. Rev. Biophys., 46, 505-529, Makarova et al., 2011, Biology Direct, 6, 38 or Wang et al., 2016, Annu. Rev. Biochem., 85, 22.1-22.38.

In yet another aspect the present invention relates to a method of monitoring a disease state comprising the performance of the method of obtaining an enriched personalized population of a target polynucleotide in a predefined interval of time. The method accordingly aims at the provision of a patient's personalized cDNA molecules to be used for the preparation of target-irrelevant sgRNAs and hence of selecting one or more uncut target polynucleotides in certain intervals. The target polynucleotides are subsequently analyzed according to their sequence and/or length etc. and they may be compared, e.g. with respect their sequence, length or other parameters, with target polynucleotides obtained previously, or with a reference polynucleotide. The repeated provision of corresponding cDNAs and, finally, of target polynucleotides allows for a tracking of molecular changes in the target polynucleotide, e.g. a gene or panel of genes. Such molecular changes may indicate the onset or presence of a disease or the absence or cure of a disease, or may have any other suitable diagnostic value. The molecular change may further allow for the adaption of therapeutic approaches, e.g. via the definition of treatment lengths, the selection of suitable medicaments, the definition of potential co-therapies etc.

In preferred embodiments, the monitoring may be performed according to any suitable consideration as to the intervals between a subject's population of mRNA molecules is used for the preparation of an enriched personalized population of a target polynucleotide. For example, any suitable interval, e.g. between hours to years may be implemented. The interval may preferably be defined in accordance with the requirements of a treatment strategy. For example, should the disease to be treated be a disease which requires a monthly or two-monthly readjustment of the therapy, the interval may accordingly be set. The subject may accordingly be stratified according to the outcome of the monitoring method.
The term "stratifying subjects" as used herein means that subjects are partitioned by a factor other than the treatment itself. This factor, may, in the present case, be the molecular change as defined herein above. The stratification may, for example, help to control confounding variables, or to facilitate the detection and interpretation between variables. Typically, the patient may be analyzed with respect to its molecular change after certain intervals. In case a certain molecular situation is encountered, or suspected, specific therapy forms or specifically adjusted therapy forms may be used.

The term disease as used herein may be any disease amenable to molecular analysis. It is preferred that the disease is cancer.

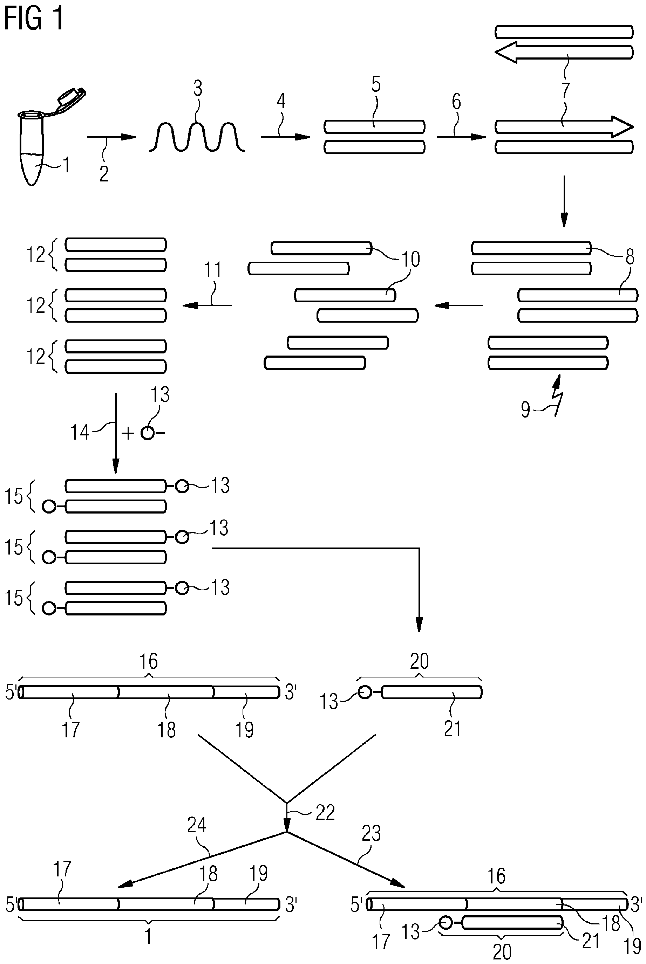

In a specific embodiments, the cancer may be breast cancer, prostate cancer, ovarian cancer, renal cancer, lung cancer, pancreas cancer, urinary bladder cancer, uterus cancer, kidney cancer, brain cancer, stomach cancer, colon cancer, melanoma or fibrosarcoma, glioblastoma or hematological leukemia or a lymphoma, e.g. a myeloid or lymphatic lymphoma. Turning now to FIGURE 1, a schematic illustration of the steps for obtaining a pool of target-irrelevant synthetic single guide RNAs (sgRNAs) according to an embodiment of the present invention is provided. In a first step a subject's sample 1 is purified 2. This step yield mRNA 3 which is reverse transcribed 4, resulting in cDNA copy of the mRNA 5. The cDNA is further amplified 6 via a polymerase chain reaction 7. The amplification yields PCR products 8, which are fragmented 9, resulting in short fragments 10 comprising inter alia sticky ends. The short fragments 10 are subsequently blunted 11 to yield blunt fragments 12. The blunt fragments 12 are connected 14 to a tag 13, preferably biotin. This yields a pool of tagged catcher oligonucleotides 15. Further, a pool of starting oligonucleotides 16 comprising a promoter segment 17, a random segment as potentially complementary sequence for the catcher oligonucleotide 18 and a binding segment 19, which is complementary to at least a portion of a scaffold sequence for interaction with the sgRNA-guided nucleic acid-binding protein is provided. The pool of starting oligonucleotides 16 is hybridized 22 with a single stranded catcher oligonucleotide 20 derived from the pool of tagged catcher oligonucleotides 15, wherein said catcher oligonucleotide comprises a tag 13 and a segment 21 which is complementary to the random segment 18. Subsequently, complexes of the starting oligonucleotide 16 and the catcher oligonucleotide 20 are removed 23 from the solution or reaction mix. Within the solution or reaction mix a reduced pool of starting oligonucleotides 24 is kept.

FIGURE 2 shows a schematic illustration of steps for the preparation of a target-irrelevant sgRNA 107 to be used according to an embodiment of the present invention. The method starts with a pool of starting oligonucleotides 16 comprising a promoter segment 17, a random segment as potentially complementary sequence for the catcher oligonucleotide 18 and a binding segment 19, which is complementary to at least a portion of a scaffold sequence for interaction with the sgRNA-guided nucleic acid-binding protein, which is hybridized 102 with a scaffold oligonucleotide 100. The subsequent reaction steps 109 take place in single tube 101. After the hybridization single stranded regions are filled in a DNA extension reaction 103 providing a double stranded DNA molecule 104. In a next step the dsDNA molecule is transcribed 106 starting via promoter activity 105. This yields an sgRNA molecule 107 comprising a sequence 18 which is not specific for a target polynucleotide according to the present invention and an sgRNA scaffold segment 108.

The figures are provided for illustrative purposes. It is thus understood that the figures are not to be construed as limiting. The skilled person in the art will clearly be able to envisage further modifications of the principles laid out herein.

### LIST OF REFERENCE NUMERALS

- 1: Subject's sample
- 2: Purification of sample
- 3: mRNA
- 4: Reverse transcription
- 5: cDNA
- 6: Amplification
- 7: Polymerase Chain Reaction (PCR)
- 8: PCR products
- 9: Fragmentation
- 10: Short fragments
- 11: Blunting
- 12: Blunt fragments
- 13: Tag
- 14: Connection to tag
- 15: Pool of tagged catcher oligonucleotides
- 16: Pool of starting oligonucleotides
- 17: Promoter segment
- 18: Random segment as potentially complementary sequence for the catcher oligonucleotide
- 19: Binding segment
- 20: Single stranded catcher oligonucleotide
- 21: Segment which is complementary to the random segment
- 22: Hybridization reaction
- 23: Removal of complex from solution
- 24: Continuation with reduced pool of starting oligonucleotides
- 100: Scaffold oligonucleotide
- 101: Single tube reaction
- 102: Hybridization reaction with scaffold oligonucleotide
- 103: DNA extension reaction
- 104: Double stranded DNA molecule
- 105: Promoter activity
- 106: Transcription reaction
- 107: Target irrelevant sgRNA molecule
- 108: sgRNA scaffold segment
- 109: Reaction steps in single tube

## Claims

1. A method of obtaining an enriched population of a target polynucleotide comprising:
(i) purifying a population of mRNA molecules from a sample obtained from a subject;
(ii) preparing cDNA from the mRNA molecules of step (i) ;
(iii) amplifying one or more target sequences from the cDNA obtained in step (ii) to obtain a pool of DNA molecules;
(iv) fragmenting the amplified DNA molecules, preferably to a size of 20 to 30 bp;
(v) connecting the fragments of step (iv) to a tag capable of binding to a cognate interactor to yield a pool of tagged catcher oligonucleotides;
(vi) providing a pool of starting oligonucleotides for the preparation of a pool of synthetic single guide RNAs synthetic single guide RNA (sgRNA) for an sgRNA-guided nucleic acid-binding protein, wherein said starting oligonucleotide comprises a promoter segment, a random segment as potentially complementary sequence for the catcher oligonucleotide and a binding segment, which is complementary to at least a portion of a scaffold sequence for interaction with the sgRNA-guided nucleic acid-binding protein;
(vii) hybridizing said pool of starting oligonucleotides and said tagged catcher oligonucleotide(s) ;
(viii) removing complexes of starting oligonucleotides and tagged catcher oligonucleotides from said pool of starting oligonucleotides by binding said tag to a cognate interactor, preferably located on a bead or a suitable surface, thereby obtaining a reduced pool of starting oligonucleotides;
(ix) preparing a pool of sgRNAs with said reduced pool of starting oligonucleotides obtained in step (viii);
(x) cleaving a mixture of polynucleotides obtained from a test sample with an sgRNA-guided nucleic acid-binding protein using the pool of sgRNAs obtained in step (ix); and
(xi) size selecting one or more uncut target polynucleotides from said mixture of polynucleotides obtained in step (x).

2. A method of obtaining a pool of personalized target-irrelevant synthetic single guide RNAs (sgRNAs) for a sgRNA-guided nucleic acid-binding protein comprising:
(i) purifying a population of mRNA molecules from a sample obtained from a subject;
(ii) preparing cDNA from the mRNA molecules of step (i) ;
(iii) amplifying one or more target sequences from the cDNA obtained in step (ii) to obtain a pool of DNA molecules;
(iv) fragmenting the amplified DNA molecules, preferably to a size of 20 to 30 bp;
(v) connecting the fragments of step (iv) to a tag capable of binding to a cognate interactor to yield a pool of tagged catcher oligonucleotides;
(vi) providing a pool of starting oligonucleotides for the preparation of a pool of synthetic single guide RNAs synthetic single guide RNA (sgRNA) for an sgRNA-guided nucleic acid-binding protein, wherein said starting oligonucleotide comprises a promoter segment, a random segment as potentially complementary sequence for the catcher oligonucleotide and a binding segment, which is complementary to at least a portion of a scaffold sequence for interaction with the sgRNA-guided nucleic acid-binding protein;
(vii) hybridizing said pool of starting oligonucleotides and said tagged catcher oligonucleotide(s) ;
(viii) removing complexes of starting oligonucleotides and tagged catcher oligonucleotides from said pool of starting oligonucleotides by binding said tag to a cognate interactor, preferably located on a bead or a suitable surface, thereby obtaining a reduced pool of starting oligonucleotides; and
(ix) preparing a pool of sgRNAs with said reduced pool of starting oligonucleotides obtained in step (viii).

3. The method of claim 1 or 2, wherein said amplification (iii) is performed as polymerase chain reaction (PCR).

4. The method of any one of claims 1 to 3, wherein said tag capable of binding to a cognate interactor is biotin and wherein said cognate interactor is streptavidin.

5. The method of claim 4, wherein said step of connecting the fragments to a biotin tag comprises an end-tailing with activated biotin, a ligation reaction with biotin or a linkage to biotin via click chemistry.

6. The method of any one of claims 1 to 5, wherein the sgRNA-guided nucleic acid-binding protein is a DNA binding Cas protein.

7. The method of claim 6, wherein the DNA binding Cas protein is a member of the family of Cas9 proteins, preferably a Cas9 protein or a derivative thereof.

8. The method of any one of claims 1 to 7, wherein said random segment comprises between about 10 to 30 random nucleotides, preferably 20 random nucleotides.

9. The method of any one of claims 1 to 8, wherein steps (vii) and (viii) are repeated 1, 2, 3, 4, 5 or more times.

10. The method of any one of claims 1 to 9, wherein said one or more target polynucleotides or target sequences represent a gene, one or more exons of a gene, and/or an open reading frame or a sub-portion thereof; or a panel of different genes, a panel of one or more exons of different genes, and/or a panel of open reading frames or sub-portions thereof, or any combination of any of the before mentioned elements.

11. The method of any one of claims 1 or 3 to 10, additionally comprising as step (xii) a step of sequencing said size selected uncut target polynucleotide(s) .

12. A kit comprising a pool of sgRNAs obtainable by the method of any one of claims 2 to 10 and a sgRNA-guided nucleic acid-binding protein, preferably a Cas9 protein or derivative thereof.

13. Use of a pool of sgRNAs obtainable by the method of any one of claims 2 to 11 for the removal of target-irrelevant polynucleotides from a mixture of polynucleotides in a Cas9-based endonuclease assay.

14. A method of monitoring a disease state comprising the performance of the method of any one of claims 1 or 3 to 11 in a predefined interval of time, preferably according to the requirements of a treatment of said disease.

15. The method of claim 14, wherein said disease is cancer.
